# EUROPEAN PATENT APPLICATION

(11) **EP 1 927 573 A1**
(43) Date of publication of application: **04.06.2008**
(21) Application number: 07380040.1
(22) Date of filing: 16.02.2007
(51) Int. Cl.: B67D 1/07, B67D 3/00

(54) **Water dispenser with a sanitisation system**

(30) Priority: 29.11.2006 ES 200603053
(71) Applicant: Canaletas, S.A., 08820 El Prat De Llobregat (Barcelona) (ES)
(72) Inventor: Morera Queral, Jordi, 08820 El Prat De Llobregat (Barcelona) (ES)
(74) Representative: Gonzalez Gonzalez, Pablo

(57) **Abstract**

This dispenser has an automatic sanitisation system that comprises:
- means of heating (3a, 3b) to heat all the water contained in the water circuit inside the dispenser when required to a sanitisation temperature of between 70 and 110 degrees centigrade; solenoid valves (13a, 13b, 13c) at the openings (12a, 12b, 12c); a duct and solenoid valve (15a, 15b) for evacuating the steam; a control circuit (4) for the means of heating (3a, 3b) the water to the sanitisation temperature and for operating the solenoid valves (13a, 13b, 13c, 15b) and any other electrical mechanisms during the sanitisation of the dispenser, all with the result that the hot water is circulated all around the circuit and through the various outlets.

## Description

### Object of the invention

This invention relates to a water dispenser fitted with a system for sanitising the dispenser.

### Background of the invention

Water dispensers are widely encountered at present in various premises such as offices, factories, schools, public gathering places, hospitals and so on. These dispensers are popular because they can dispense cool or ambient-temperature water, and some can even heat water to a service temperature of approximately 90 degrees centigrade.

An example of such dispensers is the type designed to accept large bottles or containers of water placed on top of the dispenser, thereby allowing mineral water to be supplied.

Such dispensers have an opening at the top into which the bottles containing the water to be dispensed are fitted, and a water circuit between that intake opening and one or more outlet openings fitted with opening and closing devices and connected to the circuit via means of cooling and/or heating the water to temperatures suitable for service or for consumption.

However, carrying out sanitisation or hygiene-maintenance work on these dispensers entails some degree of manual intervention by one method or another. The result is that the intervals between such sanitisation interventions tend to become lengthy owing to the cost involved, the risk of bacteriological contamination thus becoming heightened. Hence the technical problem posed is to develop a water dispenser that can implement appropriate sanitisation procedures at set intervals in a fully automatic manner, with no need for manual intervention.

### Description of the invention

The water dispenser of this invention features technical features intended to improve the operation and hygiene performance of such dispensers, arranged so that the sanitisation process can be carried out in a fully automatic and fully autonomous manner.

Thus the dispenser of this invention features an automatic sanitisation device that comprises:
- means for heating all the water contained in the water circuit inside the dispenser when required to a sanitisation temperature of between 70 and 110 degrees centigrade;
- solenoid valves making up the means for opening and closing the various nozzles or outlets from the water circuit;
- a steam-evacuation duct connected to the water circuit and fitted with an opening and closing solenoid valve; and
- a programmable control circuit intended for controlling the means of heating the water to the sanitisation temperature and the opening and closing of the water-circuit solenoid valves, the water outlets and the steam-evacuation duct during the dispenser-sanitisation process in order to make the water that has been heated to the sanitisation temperature circulate all around the water circuit and through the various water outlets.

The water circuit generally has at least one storage tank containing water to be dispensed. The means for heating the water to the sanitisation temperature are arranged in such a way as to comprise an electrical resistor inside or outside that tank, thereby achieving sanitisation from a centralised point in the dispenser. In an alternative presentation, the dispenser may feature one storage tank for supplying cold water and another for supplying hot water. In this case, the resistor employed in the storage tank for supplying hot water can also be utilised for attaining the sanitisation temperature for the water contained everywhere in the circuit.

For that purpose, it is envisaged that a water supply duct and a return duct are arranged between the two tanks; each of the ducts having its own solenoid valves, thus enabling the hot water to be circulated. These solenoid valves are likewise controlled by the programmable control circuit.

It is intended that the entire water circuit be made of a non-contaminating material that can withstand temperatures higher than the sanitisation temperature - preferably stainless steel, which is a food-grade material not requiring prior treatment to assure its durability.

In this dispenser, the sanitisation method features essentially:
- the heating of the water contained in the dispenser's water circuit to a sanitisation temperature of 70 degrees centigrade or more for a preset time, this hearing causing the destruction of pathogenic bacteria and micro-organisms of all kinds;
- the controlled opening and closing of the solenoid valves associated with said water circuit in order to make the water that has been heated to the sanitisation temperature circulate all around the circuit, thereby ensuring that the sanitising hot water reaches every spot that might pose hygiene problems;
- the discharge of part of the heated water through the various outlets, in order to sanitise the associated outlet conduits and
- the switching off of the heating devices to allow the water contained in the water circuit to fall to ambient temperature or the appropriate service temperature, the dispenser thus returning to its conventional operating status.

The programmable control circuit enables this process to be performed at times when the dispenser is not in use, e.g. at night, or at times when the premises in which the dispenser stands are closed to the public. Moreover, this control circuit can be programmed so that the sanitisation process is carried out regularly, at the intervals desired, and without the intervention of any person. The control circuit can also be programmed so that the process is performed whenever the dispenser is turned on, before it goes into its standard operating mode, as a safeguard against the dispenser having remained inactive with stagnant water inside.

### Description of the figures

To supplement the description provided and facilitate the understanding of the characteristics of this invention, a set of drawings is included with this description, and in them, for illustrative purposes and not implying any restriction, the following are shown:

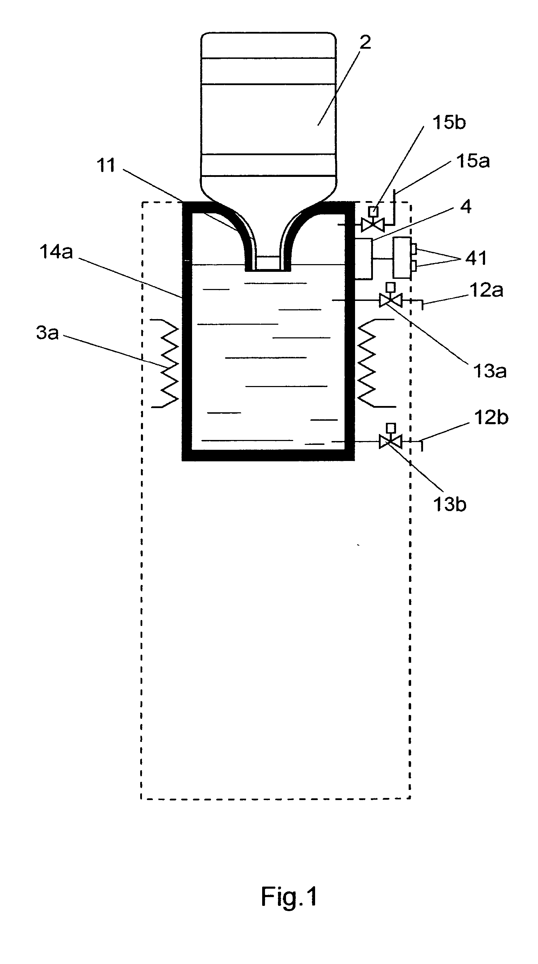

- Figure 1 shows a diagram of the dispenser with a single cold-water-production storage tank.
- Figure 2 shows a diagram of the dispenser with a cold-water production storage tank and a hot-water-production storage tank.

### Preferred embodiment of the invention

As can be seen in the above-mentioned figures, the dispenser comprises an outer housing with an opening (11) for accepting bottles (2) containing the water to be dispensed, and a water circuit between that inlet opening (11) and one or more outlet openings (12a, 12b) fitted with solenoid valves (13a, 13b) as the shut-off means for dispensing the ambient and cold water. This water circuit includes a storage tank (14a) to store the water at the desired temperature.

Positioned around or inside the storage tank (14a) are electrical resistors (3a) for arranging the sanitisation by heating the water contained inside to a temperature of between 70 and 110 degrees centigrade. The storage tank (14a) has a duct (15a) at the top for evacuating the steam that is generated, the duct being fitted with a solenoid valve (15b) that opens and closes to prevent overpressure building up inside. The dispenser has a programmable control circuit (4), which is associated with all the solenoid valves (13a, 13b, 13c, 15b), the electrical resistors (3a) for the sanitisation, and any other electrical device in the dispenser, to enable the dispenser to be used in the conventional way by any user operating its controls (41) or to carry out automatic sanitisation in accordance with a scheduled automatic programme.

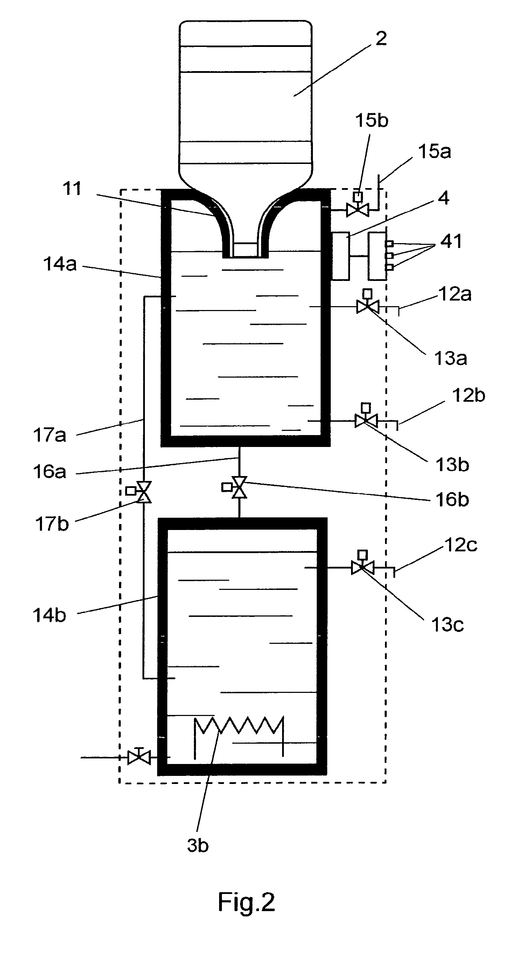

Figure 2 shows an alternative embodiment in which the dispenser can dispense hot water or service-temperature water. In this case, the water circuit includes a second storage tank (14b) for producing and storing the hot water to be dispensed by way of an additional outlet (12c), which has its own solenoid valve (13c). Positioned inside or outside that storage tank (14b) is a heating element (3b) generating the service-temperature heat and also providing for the implementation of the sanitisation by heating the water to a temperature of over 70 degrees centigrade. This storage tank (14b) is associated with the main storage tank (14a) by means of a water send conduit and a water return conduit (16a, 17a), each with its own solenoid valves (16b, 17b) which, by means of the programmable control circuit (4), implement either the filling of the second storage tank (14b) when the dispenser is being used in the conventional way, or the circulating of the water at high temperature from the storage tank (14b) to the rest of the circuit during sanitisation.

Having thus given a sufficient description of the nature of the invention, and an example of a preferred embodiment, it is stated for all pertinent purposes that the materials, shape, size and layout of the elements described may be changed provided this does not affect the essential characteristics of the invention as claimed hereunder.

## Claims

1. A water dispenser of the type having an opening (11) for accepting the bottles (2) containing the water to be dispensed, and a water circuit between that inlet opening (11) and one or more outlet openings (12a, 12b, 12c) fitted with means for opening and closing them and connected to said water circuit via means for cooling and/or heating the water to temperatures suitable for service or consumption; **characterised in that** it features an automatic sanitisation system that includes:
- means of heating (3a, 3b) for heating up all the water contained in the water circuit inside the dispenser when required to a sanitisation temperature of between 70 and 110 degrees centigrade;
- solenoid valves (13a, 13b, 13c) associated with the means of opening and closing the various outlet openings (12a, 12b, 12c) in the water circuit;
- a duct (15a) for evacuating steam, connected to the water circuit, and fitted with a solenoid valve (15b) for opening and closing it;
- a programmable control circuit (4) intended to control the means used (3a, 3b) for heating the water to the sanitisation temperature, the opening and closing of the solenoid valves (13a, 13b, 13c, 15b) for the water circuit, the outlet openings for the water (12a, 12b, 12c) and for the steam-evacuation duct (15a) during the sanitisation process for the dispenser, and also any other electrical device present in the dispenser, with the result that the water that has been heated to the sanitisation temperature is circulated all around the water circuit and through the various water outlets.

2. A dispenser, as claimed in claim 1, **characterised in that** it features water supply ducts and water return ducts (16a, 17a) with their own solenoid valves (16b, 17b), controlled by the programmable control circuit (4), to facilitate the passage of the sanitisation water between the storage tanks (14a, 14b) in the water circuit and during conventional use of the dispenser.

3. A dispenser, as claimed in claim 1, **characterised in that** the water circuit is made solely from a non-contaminating material that can withstand temperatures higher than the sanitisation temperature, preferably stainless steel.

4. A system for sanitising the dispenser as claimed in the preceding claims, **characterised in that** it comprises: the heating of the water contained in the dispenser's water circuit to a sanitisation temperature of 70 degrees or more, the opening and closing of the solenoid valves (13a, 13b, 13c, 15b) fitted in the water circuit to make the water that has been heated to a sanitisation temperature circulate all around the inside of the circuit, while discharging some of the heated water out through the various openings (13a, 13b, 13c, 15b) and outlets to arrange the sanitisation of the various outlet ducts, and the switching off of the means used for heating to allow the water contained in the water circuit to fall to ambient temperature or a suitable service temperature.
